# EUROPEAN PATENT SPECIFICATION

(11) **EP 3 498 227 B1**
(45) Date of publication and mention of the grant of the patent: **04.11.2020**
(21) Application number: 17306747.1
(22) Date of filing: 12.12.2017
(51) Int. Cl.: A61F 2/28, A61F 2/40

(54) **SHOULDER PROSTHESIS COMPONENT, SUCH AS A HUMERAL COMPONENT OR A GLENOID COMPONENT, FOR A PATIENT, AND METHOD FOR PRODUCING SUCH A SHOULDER PROSTHESIS COMPONENT FOR A PATIENT**
SCHULTERPROTHESENKOMPONENTE WIE ETWA EINE HUMERUSKOMPONENTE ODER EINE GLENOIDKOMPONENTE FÜR EINEN PATIENTEN UND VERFAHREN ZUR HERSTELLUNG SOLCH EINER SCHULTERPROTHESENKOMPONENTE FÜR EINEN PATIENTEN
COMPOSANT DE PROTHÈSE D'ÉPAULE, TEL QU'UN COMPOSANT HUMÉRAL OU UN COMPOSANT GLÉNOÏDE, POUR UN PATIENT ET PROCÉDÉ DE FABRICATION D'UN TEL COMPOSANT DE PROTHÈSE D'ÉPAULE POUR UN PATIENT

(43) Date of publication of application: 19.06.2019
(73) Proprietor: TORNIER, 38330 Montbonnot-Saint-Martin (FR)
(72) Inventor: BOUX DE CASSON, François, 38700 Le Sappey-en-Chartreuse (FR); CARDON, Jean-Emmanuel, 38420 Domène (FR); LABOULFIE, Florian, 38240 Meylan (FR); NIECHEL, Nicolas Renaud, 38700 Le Sappey-en-Chartreuse (FR)
(74) Representative: Lavoix

(56) References cited:
- EP-A1- 3 222 253
- WO-A2-2006/121330
- US-A- 5 554 190
- US-A1- 2007 203 584
- US-A1- 2011 035 014
- US-A1- 2011 160 867

## Description

The present invention relates to a shoulder prosthesis component for a patient. The invention also relates to a method for producing a shoulder prosthesis component for a patient.

In a healthy human shoulder, the head of the humerus, which is generally ball-shaped, and the glenoid cavity of the scapula articulate with each other and form a ball-and-socket joint. Total shoulder arthroplasty is a common treatment for shoulder pain resulting from arthritis or injury and leads to replace the ball-and-socket joint by a shoulder orthopedic prosthesis.

Such a shoulder prosthesis generally includes both a glenoid component to be fixedly implanted on the glenoid of the scapula and a humeral component to be fixedly implanted on the humerus, the glenoid component and the humeral component being provided to articulate directly with each other.

Over the last few years, it is proposed to design the humeral and glenoid components specifically to the patient to be treated. For this purpose, images of the humerus and of the glenoid are first acquired and the side of the humeral or glenoid component, intended to be applied directly on the humerus or glenoid is then designed to match accurate bone anatomy of the humerus or glenoid of the patient: in other words, the aforesaid side of the shoulder prosthesis component is the negative image of the humerus or glenoid. In that way, the shoulder prosthesis component is implanted without removing bone material from the humerus or glenoid, which enables to optimize preservation of the osseous capital for the patient in question. However, the geometrical congruence between the shoulder prosthesis component and the humerus or glenoid may lead to devitalize at least some parts of the bone material covered by the component because of insufficient mechanical stress on these parts. In other words, this technique based on the negative image of the humerus or glenoid may induce osteolysis for the humerus or glenoid. Besides, some securement problems may also occur when the bone material that is complementary covered by the component has a poor quality.

For example, US 5 554 190 discloses a shoulder prosthesis component that comprises a body, namely a shaft, defining an implantation axis. This prosthetic body is designed to be received within a long bone, such as the humerus. US 5 554 190 provides that the shape of the prosthetic body is adapted to the shape and bone density of the long bone of a given patient. For this purpose, images corresponding to cross-sections distributed over the length of the long bone and resulting for example from CT scans are used to produce a model for the shape and the bone density of the patient's bone. Then this model is used to adjust the shape of the prosthetic body, especially with adapting the shape and the size of the cross-section of this prosthetic body along the implantation axis, so that the cross-section of the prosthetic component is reduced by at least 1% with respect to the inner cross-section of the patient's bone. Thus, US 5 554 190 teaches to design the prosthetic shaft so that the latter fits ideally into the patient's bone.

One of the goals of the invention is to propose an improved shoulder prosthesis component for a patient, which in particular is lifelong and stable on the humerus or glenoid after implantation.

To this end, one object of the invention is a shoulder prosthesis component for a patient, as defined in claim 1.

Another object of the invention is a method for producing a shoulder prosthesis component for a patient, especially for producing the shoulder prosthesis component according to claim 1, this method being as defined in claim 11.

After implantation of the shoulder prosthesis component according to the invention on a bone of a patient, especially on a humerus or a glenoid of the patient, the cancellous bone internal layer of this bone is compacted within the bone by the implantation body of the component in a manner that is differentiated along and around a longitudinal axis of the bone. Furthermore, the manner according to which the cancellous bone internal layer is compacted is planned in function of preoperative local bone density and quantity of the cancellous bone internal layer. In that way, the implantation body of the component according to the invention is designed to compact, in other words is designed to deform the cancellous bone internal layer so as to produce a predicted result for the compaction of the bone material in the bone when the component is inserted into this bone. Thanks to the invention, an appropriate mechanical stress having a predetermined value that is both non zero and not too high can be applied by the implantation body of the component on the bone material in the bone, being noted that the predetermined value of this mechanical stress may be differentiated along and/or around the longitudinal axis of the bone according to the aforesaid planned manner: this mechanical stress stimulates the bone material in the bone and thus avoids any devitalization or even osteolysis. Furthermore, even if the cancellous bone internal layer has a poor quality, the invention enables to take this into account for designing the implantation body of the component, especially by adjusting the localization and amount of the compression performed by the implantation body, and thus to avoid any lack of securement for the implantation body into the bone.

Additional advantageous features for the shoulder prosthesis component and for the method for producing a shoulder prosthesis component according to the invention are specified in the other claims.

Embodiments of the invention will be better understood from reading the description which will follow, which is given solely by way of example and with reference to the drawings, in which:
- figure 1 is a schematic view of a humerus of a patient;
- figure 2 is a schematic view of a humeral component that is implanted at the humerus of figure 1;
- the figures 3 and 4 are views similar to figure 2, respectively illustrating two other humeral components; and
- figure 5 is a view similar to figure 2, illustrating a glenoid component that is implanted at a glenoid of a patient.

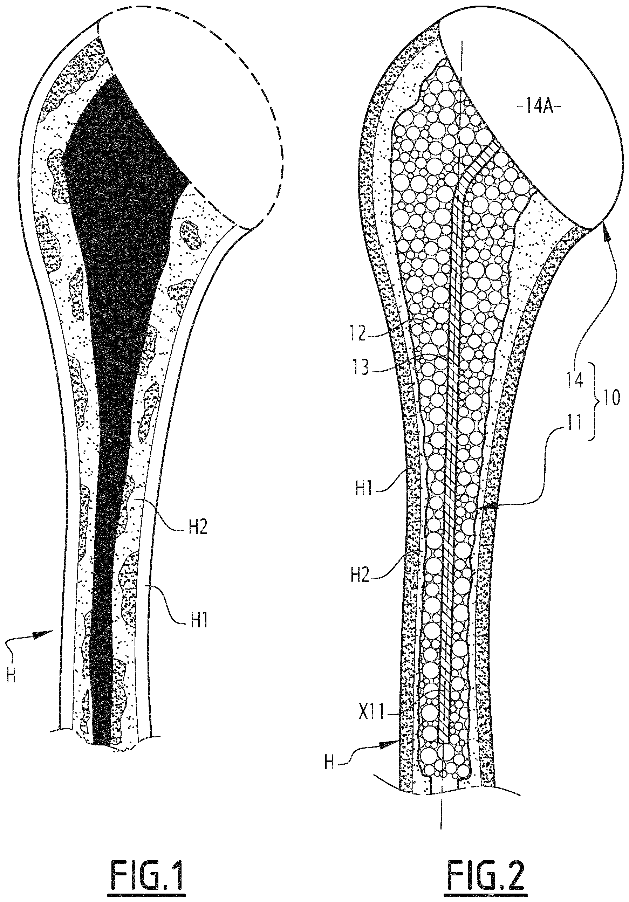

Figure 1 shows a human humerus H intended to be prosthesized with a humeral component. More precisely, in figure 1 the humerus H is depicted as this humerus appears in one of the CT images of a CT scan performed on the humerus of a patient before implanting the aforesaid humeral component. The CT image corresponding to figure 1 allows distinguishing two different bone layers of the humerus H, especially for the diaphyseal and metaphyseal parts of the latter, i.e.:
- an external bone layer H1 that is made of hard bone and that corresponds to a cortical bone layer, and
- an internal bone layer H2 that is made of soft bone and that corresponds to a cancellous or spongious bone layer.

The cortical bone external layer H1 forms an outer sleeve for the humerus H, especially for the diaphyseal and metaphyseal parts of the humerus, and is internally covered by the cancellous bone internal layer H2 which in turn internally defines a free volume that corresponds to a medullary canal of the humerus H. It is important to note that the accurate shape of the cortical bone external layer H1 and the accurate shape of the cancellous bone internal layer H2 are specific to the patient to be treated. In particular, the cancellous bone internal layer H2 has bone density and quantity which vary both along and around a longitudinal axis of the humerus H, the corresponding local variations of bone density and quantity of the cancellous bone internal layer H2 being specific to the patient in question. For example, the cancellous internal bone layer H2 may include a first portion, which is located both at a first level along the longitudinal axis of the humerus H and at a first angle around this longitudinal axis and which has a bone density and a bone quantity, i.e. a bone thickness, that are different from the bone density and thickness of a second portion of the cancellous bone internal layer H2, which is located both at a second level along the longitudinal axis of the humerus and at a second angle around this longitudinal axis, the second level being different from the first level and/or the second angle being different from the first angle, it being understood that the first and second levels and the first and second angles have any respective values depending on the patient..

In view of producing a humeral component to be implanted at the humerus H of the patient in question, the local bone density and quantity of the cancellous bone internal layer H2 of the humerus H are quantified, especially for the diaphyseal part and for the metaphyseal part of the humerus. As the head of the humerus H is intended to be at least partly removed during surgery, the bone anatomy of this head has not be characterized, which explains why the head of the humerus H is drawn only in dotted lines in figure 1. In practice, the technique used to perform such a quantification of bone density and quantity of the cancellous bone internal layer H2 of the humerus H is not limitative provided that this technique provides patient bone data representative of preoperative local bone density and quantity of the cancellous bone internal layer H2.

In some embodiments, these patient bone data result from segmenting CT images of a CT scan performed on the humerus H. As indicated above, an example of such a CT image is shown in figure 1. In such CT images, the bone density may be characterized in Hounsfield units (HU), as well known in the field of medical imaging technologies: in the example of figure 1, the part of the humerus H having the highest bone density, in other words the part of the humerus having a value in Hounsfield units that is higher than a threshold, appears as white and corresponds to the cortical bone external layer H1; the part of the humerus having a zero bone density appears as black and corresponds to the medullary canal of the humerus H; the other portions of the humerus H appear as darker or lighter grey and correspond to the cancellous bone internal layer H2 which has bone density whose local values are both greater than zero and lower than the bone density value of the cortical bone external layer H1. Furthermore, in such CT images, the bone quantity may be characterized in dimension units, such as millimeters, used to quantify the thickness of the cancellous bone internal layer H2. Of course, rather than considering two dimensional CT images as the one shown in figure 1, three dimensional CT images may be considered, on the understanding that using and segmenting such two dimensional images and/or three dimensional CT images depend on capability of the imaging installation, especially the software and hardware thereof, that is used.

In some embodiments, other imaging techniques may be used as an alternative or supplement to a CT scan in order to provide the aforesaid patient bone data. For example, X-ray images and/or MRI images may be performed on the humerus H and analyzed, typically by computer, in order to provide the aforesaid patient bone data. In any case, these data are preoperative, that is to say representative of the bone anatomy of the humerus H of the patient before this humerus is surgically treated in view of receiving a humeral component.

Also before surgically treating the humerus H, the aforesaid patient bone data are used to design an implantation body of a humeral component that is to be produced and intended to be implanted at the humerus during a future surgery. As explained later in reference to the figures 2 to 4, various embodiments are possible for this implantation body. In any case, the design of this implantation body is based on the aforesaid patient bone data so that the humerus H as prospectively prosthesized with the humeral component to be produced receives inwardly the implantation body and has its cancellous bone internal layer H2 that is compacted in a planned manner that is differentiated along and around an implantation axis of the implantation body depending on preoperative local bone density and quantity of this cancellous bone internal layer H2.

In practice, one possibility is to use rules to design this implantation body, these rules being based on the patient bone data and relating preoperative local bone density and quantity of the cancellous bone internal layer H2 to a desired compacted condition for this cancellous bone internal layer of the humerus H as prosthesized with the humeral component to be produced. The particulars of such rules are not limitative. For example, one of these rules is to plan that a portion of the cancellous bone internal layer H2, having a low density and a high thickness has to be compacted with a high rate of compaction, for example around 50%. Another rule may be to plan that a portion of this cancellous internal bone layer H2, having a low bone density and a small thickness has to be compacted with a low rate of compaction, for example less than 50%, such as around 25%. Other rules may be contemplated. Besides, instead of using rules with compaction rates, the rules may be with compaction values.

Other possibilities for operating the design of the implantation body on the basis of the aforesaid patient bone data may be considered. More generally, it is important to understand that the implantation body is designed to apply stresses on the cancellous bone internal layer H2 and thus to deform and compact this layer H2 so as to produce a predicted result for the compaction of the various portions of that bone material in the humerus H when in the future this implantation body is inserted into the humerus. In some embodiments, designing the implantation body provides to design a peripheral part of this implantation body so that this peripheral part is shaped and structured in order to compact the cancellous internal bone layer H2 in the aforesaid planned manner. In some embodiments, the compaction effect provided by such a peripheral part of the implantation body may also take into account the fact that this peripheral part is deformed while compacting the cancellous bone internal layer H2 in the aforesaid planned manner, the implantation body being designed so that its peripheral part is shaped and structured accordingly.

Once the design of the implantation body of the humeral component to be produced is established, a following step is to manufacture this designed implantation body. The manufacturing techniques which can be used are not limitative. In some embodiments, an additive manufacturing process may be used.

Turning now to figure 2, a humeral component 10 is considered as a first embodiment of a humeral component produced by the method described up to here.

The humeral component 10 comprises an implantation body 11 that is designed as described above. The implantation body 11 defines an implantation axis X11 intended to be aligned with the longitudinal axis of the humerus H upon implantation of the humeral component 10 at the humerus H. As discussed above in connection with designing an implantation body on the basis of the aforesaid patient bone data representative of preoperative local bone density and quantity of the cancellous bone internal layer H2, the implantation body 11 of the humeral component 10 is designed to be received within the humerus H and to compact therein the cancellous bone internal layer H2 in a planned manner that is differentiated along and around the implantation axis X11 depending on preoperative local bone density and quantity of this cancellous bone internal layer H2: as shown in figure 2 in which the humerus is drawn as prosthesized by the humeral component 10, the implantation body 11 is received within the humerus H and the cancellous bone internal layer H2 is compacted by the implantation body 11 in the aforesaid planned manner. For example, one or more portions of the cancellous bone internal layer H2 are compacted with a compaction rate of around 50% (or with a given compaction value) and/or one or more other portions of this layer H2 are compacted with a higher compaction rate (or with a higher compaction value) and/or one or more other portions of this layer H2 are compacted with a lower compaction rate (or with a lower compaction value), depending on the preoperative bone density and quantity of each of these various portions that are located at different respective levels along the implantation axis X11 and/or at different respective angles around the implantation axis X11.

In some embodiments as the one shown in figure 2, the implantation body 11 includes a peripheral part 12 and a central core 13. The central core 13, which may be a metallic rod for example, is more rigid than the peripheral part 12, so as to support this peripheral part 12. Upon implantation of the humeral component 10, the peripheral part 12 is intended to be in contact with the cancellous bone internal layer H2 and is accordingly shaped and structured to compact this layer H2 in the aforesaid planned manner. In practice, the peripheral part 12 may be shaped and structured to be deformed while compacting the cancellous bone internal layer H2 in the aforesaid planned manner. In the example shown in figure 2, the peripheral part 12 comprises or even is made of a porous structure for that purpose: this porous structure is provided to have a porosity that is varied along and around the implantation axis X11 depending on preoperative local bone density and quantity of the cancellous bone internal layer H2. In that way, upon implantation of the implantation body 11 in the humerus H, the porous structure of the peripheral part 12 combines a mechanical stiffness for pressing the layer H2 and a deformation capacity for adapting its shape and thus avoiding any overstress on the layer H2: in doing so, the porous structure of the peripheral part 12 can compact the cancellous bone internal layer H2 with a predicted compaction rate that is varied along and around the implantation axis X11 in accordance with the aforesaid planned manner with which the various portions of the layer H2 are desired to be compacted. In practice, such a porous structure of the peripheral part 12 may be manufactured by additive manufacturing processes.

In some embodiments as the one shown in figure 2, the humeral component 10 further comprises an articulation head 14. The articulation head 14 is provided with an articulation surface 14A intended to articulate either with a glenoid prosthetic component implanted at the glenoid of the patient or directly with the glenoid of the patient, in both cases so as to restore shoulder joint for the patient. The articulation head 14 is also provided to be coupled with the implantation body 11, in particular with the central core 13 of the latter: in practice, the corresponding coupling arrangements are not limitative and will not be further described insofar as various solutions are available in the art. The other particulars of the articulation head 14 are not limitative.

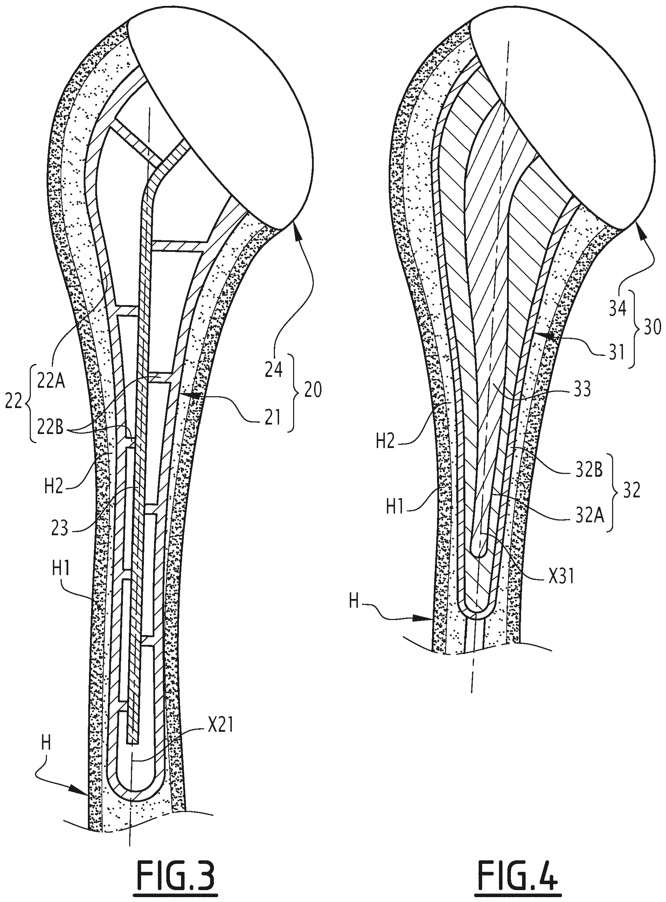

Turing now to figure 3, a humeral component 20 is considered as a second embodiment of a humeral component produced by the method described above.

The humeral component 20 comprises an implantation body 21 and an articulation head 24, which are functionally similar to the implantation body 11 and the articulation head 14 of the humeral component 10 respectively. The implantation body 21 defines an implantation axis X21 that is similar to the implantation axis X11 for the implantation body 12 of the humeral component 10. Moreover, the implantation body 21 includes a peripheral part 22 and a central core 23, which are functionally similar to the peripheral part 12 and the central core 13 of the implantation body 11 respectively.

The peripheral part 22 of the implantation body 21 is distinguished by its structure, in the sense that the peripheral part 22 comprises or even consists of an elastic shell 22A and spacers 22B that mechanically connect the elastic shell 22A to the central core 23. The elastic shell 22A has a deformation capability that is varied along and around the implantation axis X21 depending on preoperative local bone density and quantity of the cancellous bone internal layer H2 of the humerus H. Upon implantation of the implantation body 21 in the humerus H, the elastic shell 22A applies on the cancellous bone internal layer H2 stresses resulting at least from the elastic material, the shape and the thickness of the elastic shell 22A and from the action of the spacers 22B on the elastic shell 22A, so that the peripheral part 22 can compact this layer H2 with a predicted compaction rate and/or value that is varied along and around the implantation axis X21 in accordance with the aforesaid planned manner with which the various portions of this layer H2 are desired to be compacted, as explained previously.

In practice, the peripheral part 22 may be manufactured by additive manufacturing processes.

Turning now to figure 4, a humeral component 30 is considered as a third embodiment of a humeral component produced by the method described above.

The humeral component 30 comprises an implantation body 31 and an articulation head 34, which are functionally similar to the implantation body 11 and the articulation head 14 of the humeral component 10 respectively. The implantation body 31 defines an implantation axis X31 that is similar to the implantation axis X11 for the implantation body 12 of the humeral component 10. Moreover, the implantation body 31 includes a peripheral part 32 and a central core 33, which are functionally similar to the peripheral part 12 and the central core 13 of the implantation body 11 respectively.

The peripheral part 32 is distinguished by its structure, in the sense that the peripheral part 32 comprises or even consists in an intermediate, soft undercoat 32A, which covers the central core 33, and an external, frangible coating 32B, which overlays the undercoat 32. Both undercoat 32A and coating 32B have a shape adaptability, that is to say that they can each adapt their shape upon implantation of the humeral component 30, the shape adaptability for each of the undercoat 32A and the coating 32B being varied along and around the implantation axis X31 depending on preoperative local bone density and quantity of the cancellous bone internal layer H2. However, these two shape adaptabilities are implemented by two different basic mechanisms: the undercoat 32A is conformable by elastic deformation whereas the coating 32B is conformable by surface breaking, on the understanding that the material of the coating 32B is bioabsorbable so that the detached particles from the coating 32B are absorbed by the tissues of the patient. This bioabsorbable material may be ceramic type and is for example a mixture of hydroxyapatite and tricalciumphosphate, or any brittle biological bone substitute. Thanks to the respective shape adaptability of the undercoat 32A and of the coating 32B, the peripheral part 32 of the implantation body 31 applies on the cancellous bone internal layer H2 some predetermined stresses upon implantation of the implantation body 31 in the humerus H, so that this peripheral part 32 can compact this layer H2 with a predicted compaction rate and/or value that is varied along and around the implantation axis X31 in accordance with the aforesaid planned manner with which the various portions of this layer H2 are desired to be compacted as explained previously.

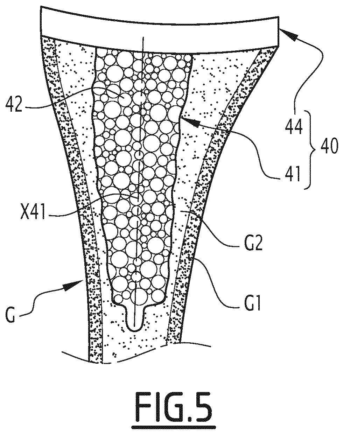

All of the above considerations relevant to the humerus of a patient and to producing a humeral component for this humerus are applicable to the glenoid of a patient and to produce a glenoid component for this glenoid. For example, figure 5 shows a glenoid G of a patient, which has a cortical bone external layer G1 and a cancellous bone internal layer G2 and which is prosthesized with a glenoid component 40. In the same way as explained previously for the humeral components 10, 20 and 30, the glenoid component 40 comprises an implantation body 41 that defines an implantation axis X41 and that is designed to compact the cancellous bone internal layer G2 in a planned manner that is differentiated along and around the implantation axis X41 depending on preoperative local bone density and quantity of this cancellous bone internal layer G2 when the glenoid component 40 is implanted at the glenoid G. In figure 5, the implantation body 41 is received within the glenoid G and the cancellous bone internal layer G2 of the glenoid G is compacted by the implantation body 41 in the aforesaid planned manner.

As for the implantation bodies 11, 21 and 31 of the humeral components 10, 20 and 30, the implantation body 41 may include a peripheral part 42 that is intended to be in contact with the cancellous bone internal layer G2, being able to apply stresses on this layer G2 by compacting the latter with a predicted compaction rate and/or value that is varied along and around the implantation axis X41 in accordance with the aforesaid planned manner with which the various portions of the layer G2 are desired to be compacted. Various embodiments for the implantation body 41 and for its peripheral part 42 may be considered. In figure 5, the peripheral part 42 is made of a porous structure as the one contemplated for the peripheral part 12 of the implantation body 11 of the humeral component 10. In alternative, the peripheral part 42 may correspond to one of the peripheral parts 22 and 32 of the implantation bodies 21 and 31.

In the embodiment of figure 5, the glenoid component 40 further comprises an articulation cup 44 which is functionally similar to the articulation heads 14, 24 and 34 of the humeral components 10, 20 and 30, except that an articulation surface of the articulation cup 44 is intended to articulate either with a humeral prosthetic component implanted at the humerus of the patient, such as one of the humeral components 10, 20 and 30, or directly with the head of the humerus of the patient, in both cases so as to restore shoulder joint.

The embodiment shown in figure 5 also illustrates that a central support such as the central cores 13, 23 and 33 disclosed above, may be omitted for the implantation body, as shown for the implantation body 41 in figure 5.

## Claims

1. A shoulder prosthesis component (10; 20; 30; 40) for a patient, comprising an implantation body (11; 21; 31; 41), which defines an implantation axis (X11; X21; X31; X41) and which is designed to be received within a bone (H; G) of the patient that includes a cortical bone external layer (H1; G1) and a cancellous bone internal layer (H2; G2), **characterized in that** the implantation body is designed to compact the cancellous bone internal layer in a planned manner that is differentiated along and around the implantation axis depending on preoperative local bone density and quantity of the cancellous bone internal layer.

2. The shoulder prosthesis component of claim 1, wherein the implantation body (11; 21; 31; 41) includes a peripheral part (12; 22; 32; 42) that is shaped and structured to compact the cancellous bone internal layer (H2; G2) in said planned manner.

3. The shoulder prosthesis component of claim 2, wherein the peripheral part (12; 22; 32; 42) is also shaped and structured to be deformed while compacting the cancellous bone internal layer (H2; G2) in said planned manner.

4. The shoulder prosthesis component of claim 2 or claim 3, wherein the peripheral part (12; 42) comprises a porous structure which has a porosity that is varied along and around the implantation axis (X11; X41) depending on preoperative local bone density and quantity of the cancellous bone internal layer (H2; G2).

5. The shoulder prosthesis component of any one of the claims 2 to 4, wherein the implantation body (11; 21; 31) further includes a central core (13; 23; 33) that is more rigid than the peripheral part.

6. The shoulder prosthesis component of claim 5, wherein the peripheral part (22) comprises an elastic shell (22A), which is supported by the central core (23) by means of spacers (22B) and which has a deformation capability that is varied along and around the implantation axis (X21) depending on preoperative local bone density and quantity of the cancellous bone internal layer (H2).

7. The shoulder prosthesis component of claim 5 or claim 6, wherein the peripheral part (32) comprises:
- an intermediate, soft undercoat (32A), which covers the central core (33) and which has a shape adaptability that is implemented by deformation and that is varied along and around the implantation axis (X31) depending on preoperative local bone density and quantity of the cancellous bone internal layer (H2), and
- an external, frangible coating (32B), which overlays the undercoat and which has a shape adaptability that is implemented by breaking and that is varied along and around the implantation axis depending on preoperative local bone density and quantity of the cancellous bone internal layer (H2).

8. The shoulder prosthesis component of any one of the preceding claims, wherein the shoulder prosthesis component (10; 20; 30; 40) further comprises an articulation member (14; 24; 34; 44), which is provided with an articulation surface (14A) and which is provided to be coupled with the implantation body (11; 21; 31; 41).

9. The shoulder prosthesis component of any one of the preceding claims, wherein the shoulder prosthesis component is a humeral component (10; 20; 30), the implantation body (11; 21; 31) being designed for the humerus (H) of the patient.

10. The shoulder prosthesis component of any one of the claims 1 to 8, wherein the shoulder prosthesis component is a glenoid component (40), the implantation body (41) being designed for the glenoid (G) of the patient.

11. A method for producing a shoulder prosthesis component (10; 20; 30; 40) for a patient, the method comprising:
- a data providing step, in which a bone (H; G) of the patient that includes a cortical bone external layer (H1; G2) and a cancellous bone internal layer (H2; G2) is **characterized by** patient bone data representative of preoperative local bone density and quantity of the cancellous bone internal layer,
- a design step, **characterized in that** an implantation body (11; 21; 31; 41) of the shoulder prosthesis component to be produced is designed from the patient bone data so that the bone as prosthesized with the shoulder prosthesis component to be produced receives inwardly the implantation body and has its cancellous bone internal layer that is compacted in a planned manner that is differentiated along and around an implantation axis of the implantation body depending on preoperative local bone density and quantity of the cancellous bone internal layer, and
- a manufacturing step, in which the implantation body designed at the design step is manufactured.

12. The method of claim 11, wherein the design step includes:
- defining rules which are based on the patient bone data and which relate preoperative local bone density and quantity of the cancellous bone internal layer (H2; G2) to a desired compacted condition for the cancellous bone internal layer of the bone as prosthesized with the shoulder prosthesis component (10; 20; 30; 40) to be produced, and
- using the rules to design the implantation body (11; 21; 31; 41).

13. The method of claim 11 or claim 12, wherein the data providing step includes performing a CT scan of the bone (H; G) and segmenting CT images of the CT scan into the patient bone data.

14. The method of any one of the claims 11 to 13, wherein the manufacturing step includes using an additive manufacturing process.

15. The method of any one of the claims 11 to 14, wherein the method further comprises coupling the implantation body (11; 21; 31; 41) manufactured at the manufacturing step with an articulation member (14; 24; 34; 44) of the shoulder prosthesis component (10; 20; 30; 40), the articulation member being provided with an articulation surface (14A).

## Patentansprüche

1. Schulterprothesenkomponente (10; 20; 30; 40) für einen Patienten, aufweisend einen Implantationskörper (11; 21; 31; 41), der eine Implantationsachse (X11; X21; X31; X41) definiert und der dazu ausgestaltet ist, in einem Knochen (H; G) des Patienten aufgenommen zu werden, der eine kortikale Knochenaußenschicht (H1; G1) und eine spongiöse Knocheninnenschicht (H2; G2) aufweist,
**dadurch gekennzeichnet, dass**
der Implantationskörper dazu ausgestaltet ist, die spongiöse Knocheninnenschicht in einer geplanten Weise zu verdichten, die entlang und um die Implantationsachse herum in Abhängigkeit von der präoperativen lokalen Knochendichte und der Menge der Spongiöse Knocheninnenschicht differenziert wird.

2. Schulterprothesenkomponente nach Anspruch 1,
wobei der Implantationskörper (11; 21; 31; 41) einen peripheren Teil (12; 22; 32; 42) aufweist, der derart geformt und gestaltet ist, dass er die spongiöse Knocheninnenschicht (H2; G2) in der geplanten Weise verdichten kann.

3. Schulterprothesenkomponente nach Anspruch 2,
wobei der periphere Teil (12; 22; 32; 42) ebenfalls derart geformt und gestaltet ist, dass er verformt werden kann, während die spongiöse Knocheninnenschicht (H2; G2) in der geplanten Weise verdichtet wird.

4. Schulterprothesenkomponente nach Anspruch 2 oder 3,
wobei der periphere Teil (12; 42) eine poröse Struktur enthält, die eine Porosität aufweist, die entlang und um die Implantationsachse (X11; X41) herum in Abhängigkeit von der präoperativen lokalen Knochendichte und der Menge der spongiösen Knocheninnenschicht (H2; G2) variiert.

5. Schulterprothesenkomponente nach einem der Ansprüche 2 bis 4,
wobei der Implantationskörper (11; 21; 31) ferner einen zentralen Kern (13; 23; 33) aufweist, der steifer als der periphere Teil ist.

6. Schulterprothesenkomponente nach Anspruch 5,
wobei der periphere Teil (22) einen elastischen Mantel (22A) aufweist, die durch den zentralen Kern (23) mittels Abstandshaltern (22B) gehaltert ist und die ein Verformungsvermögen aufweist, das entlang und um die Implantationsachse (X21) in Abhängigkeit von der präoperativen lokalen Knochendichte und der Menge der Spongiöse Knocheninnenschicht(H2) variiert.

7. Schulterprothesenkomponente nach Anspruch 5 oder 6,
wobei der periphere Teil (32) folgendes aufweist:
- eine weiche Zwischen-Unterschicht (32A), die den zentralen Kern (33) bedeckt und die eine Formanpassungsfähigkeit aufweist, die durch Verformung realisiert wird und die entlang und um die Implantationsachse (X31) in Abhängigkeit von der präoperativen lokalen Knochendichte und der Menge der spongiösen Knocheninnenschicht (H2) variiert, und
- eine äußere, zerbrechliche Beschichtung (32B), die über der Unterschicht liegt und eine Formanpassungsfähigkeit aufweist, die durch Brechen realisiert wird und die entlang und um die Implantationsachse herum in Abhängigkeit von der präoperativen lokalen Knochendichte und der Menge der Spongiosa-Innenschicht (H2) variiert.

8. Schulterprothesenkomponente eines der vorstehenden Ansprüche,
wobei die Schulterprothesenkomponente (10; 20; 30; 40) ferner ein Gelenkelement (14; 24; 34; 44) aufweist, das mit einer Gelenkfläche (14A) versehen ist und das dazu vorgesehen ist, mit dem Implantationskörper (11; 21; 31; 41) gekoppelt zu werden.

9. Schulterprothesenkomponente nach einem der vorstehenden Ansprüche,
wobei die Schulterprothesenkomponente eine Humeruskomponente (10; 20; 30) ist, wobei der Implantationskörper (11; 21; 31) für den Humerus (H) des Patienten ausgelegt ist.

10. Schulterprothesenkomponente nach einem der Ansprüche 1 bis 8,
wobei die Schulterprothesenkomponente eine Glenoidkomponente (40) ist, wobei der Implantationskörper (41) für das Glenoid (G) des Patienten ausgelegt ist.

11. Verfahren zur Herstellung einer Schulterprothesenkomponente (10; 20; 30; 40) für einen Patienten, wobei das Verfahren folgendes umfasst:
- einen Datenbereitstellungsschritt, bei dem ein Knochen (H; G) des Patienten, der eine kortikale Knochenaußenschicht (H1; G2) und eine spongiöse Knocheninnenschicht (H2; G2) aufweist, durch Patientenknochendaten gekennzeichnet wird, die für die präoperative lokale Knochendichte und die Menge der spongiösen Knocheninnenschicht repräsentativ sind,
- einen Gestaltungsschritt, **dadurch gekennzeichnet, dass** ein Implantationskörper (11; 21; 31; 41) der herzustellenden Schulterprothesenkomponente aus den Patientenknochendaten derart gestaltet wird, dass der mit der herzustellenden Schulterprothesenkomponente prothetisch hergestellte Knochen den Implantationskörper nach innen aufnimmt und seine spongiöse Knocheninnenschicht aufweist, die in einer geplanten Weise verdichtet wird, die entlang und um eine Implantationsachse des Implantationskörpers in Abhängigkeit von der präoperativen lokalen Knochendichte und der Menge der spongiöse Knocheninnenschicht differenziert wird, und
- einen Herstellungsschritt, in dem der im Gestaltungsschritt entworfene Implantationskörper hergestellt wird.

12. Verfahren nach Anspruch 11,
wobei der Gestaltungsschritt folgendes umfasst:
- Definieren von Regeln, die auf den Patientenknochendaten basieren und die die präoperative lokale Knochendichte und Menge der spongiösen Knocheninnenschicht (H2; G2) mit einem gewünschten verdichteten Zustand für die spongiöse Knocheninnenschicht des Knochens, wie er mit der herzustellenden Schulterprothesenkomponente (10; 20; 30; 40) prothetisiert wird, in Beziehung setzen, und
- Anwenden der Regeln zum Gestalten des Implantationskörpers (11; 21; 31; 41).

13. Verfahren nach Anspruch 11 oder 12,
wobei der Datenbereitstellungsschritt das Durchführen eines CT-Scans des Knochens (H; G) und das Segmentieren von CT-Bildern des CT-Scans in die Patientenknochendaten umfasst.

14. Verfahren nach einem der Ansprüche 11 bis 13,
wobei der Herstellungsschritt das Verwenden eines additiven Herstellungsverfahrens umfasst.

15. Verfahren nach einem der Ansprüche 11 bis 14,
wobei das Verfahren ferner das Koppeln des im Herstellungsschritt hergestellten Implantationskörpers (11; 21; 31; 41) mit einem Gelenkelement (14; 24; 34; 44) der Schulterprothesenkomponente (10; 20; 30; 40) umfasst, wobei das Gelenkelement mit einer Gelenkfläche (14A) versehen ist.

## Revendications

1. Composant de prothèse d'épaule (10 ; 20 ; 30 ; 40) pour un patient, comprenant un corps d'implantation (11 ; 21 ; 31 ; 41) qui définit un axe d'implantation (X11 ; X21 ; X31 ; X41) et qui est conçu pour être reçu à l'intérieur d'un os (H ; G) du patient qui comprend une couche externe d'os cortical (H1 ; G1) et une couche interne d'os spongieux (H2 ; G2), **caractérisé en ce que** le corps d'implantation est conçu pour compacter la couche interne d'os spongieux d'une manière planifiée qui est différenciée le long et autour de l'axe d'implantation en fonction de la densité osseuse locale préopératoire et de la quantité de la couche interne d'os spongieux.

2. Composant de prothèse d'épaule selon la revendication 1, dans lequel le corps d'implantation (11 ; 21 ; 31 ; 41) comprend une partie périphérique (12 ; 22 ; 32 ; 42) qui est formée et structurée pour compacter la couche interne d'os spongieux (H2 ; G2) selon ladite manière planifiée.

3. Composant de prothèse d'épaule selon la revendication 2, dans lequel la partie périphérique (12 ; 22 ; 32 ; 42) est également formée et structurée pour être déformée tout en compactant la couche interne d'os spongieux (H2 ; G2) selon ladite manière planifiée.

4. Composant de prothèse d'épaule selon la revendication 1 ou la revendication 3, dans lequel la partie périphérique (12 ; 42) comprend une structure poreuse qui a une porosité qui est modifiée le long et autour de l'axe d'implantation (X11 ; X41) en fonction de la densité et de la quantité d'os local préopératoire de la couche interne d'os spongieux (H2 ; G2).

5. Composant de prothèse d'épaule selon l'une quelconque des revendications 2 à 4, dans lequel le corps d'implantation (11 ; 21 ; 31) comprend en outre un noyau central (13 ; 23 ; 33) qui est plus rigide que la partie périphérique.

6. Composant de prothèse d'épaule selon la revendication 5, dans lequel la partie périphérique (22) comprend une coque élastique (22A) qui est supportée par le noyau central (23) au moyen de dispositifs d'espacement (22B) et qui a une capacité de déformation qui est modifiée le long et autour de l'axe d'implantation (X21) en fonction de la densité et de la quantité d'os local préopératoire de la couche interne d'os spongieux (H2).

7. Composant de prothèse d'épaule selon la revendication 5 ou la revendication 6, dans lequel la partie périphérique (32) comprend :
une sous-couche souple intermédiaire (32A) qui recouvre le noyau central (33) et qui a une adaptabilité de forme qui est mise en oeuvre par déformation et qui est modifiée le long et autour de l'axe d'implantation (X31) en fonction de la densité et de la quantité d'os local préopératoire de la couche interne d'os spongieux (H2), et
un revêtement cassant externe (32B) qui recouvre la sous-couche et qui a une adaptabilité de forme qui est mise en oeuvre par fracture et qui est modifiée le long et autour de l'axe d'implantation en fonction de la densité et de la quantité d'os local préopératoire de la couche interne d'os spongieux (H2).

8. Composant de prothèse d'épaule selon l'une quelconque des revendications précédentes, dans lequel le composant de prothèse d'épaulement (10 ; 20 ; 30 ; 40) comprend en outre un élément d'articulation (14 ; 24 ; 34 ; 44) qui est prévu avec une surface d'articulation (14A) et qui est prévu pour être couplé avec le corps d'implantation (11 ; 21 ; 31 ; 41).

9. Composant de prothèse d'épaule selon l'une quelconque des revendications précédentes, dans lequel le composant de prothèse d'épaulement est un composant huméral (10 ; 20 ; 30), le corps d'implantation (11 ; 21 ; 31) étant conçu pour l'humérus (H) du patient.

10. Composant de prothèse d'épaule selon l'une quelconque des revendications 1 à 8, dans lequel le composant de prothèse d'épaulement est un composant glénoïde (40), le corps d'implantation (41) étant conçu pour le glénoïde (G) du patient.

11. Procédé pour produire un composant de prothèse d'épaulement (10 ; 20 ; 30 ; 40) pour un patient, le procédé comprenant :
une étape de fourniture de données, dans laquelle un os (H ; G) du patient qui comprend une couche d'externe d'os cortical (H1 ; G2) et une couche interne d'os spongieux (H2 ; G2) est **caractérisé par** des données osseuses de patient représentatives de la densité et de la quantité d'os local préopératoire de la couche interne d'os spongieux ;
une étape de conception **caractérisée en ce qu'**un corps d'implantation (11 ; 21 ; 31 ; 41) du composant de prothèse d'épaule à produire est conçu d'après les données osseuses du patient de sorte que l'os, lorsqu'il est remplacé avec le composant de prothèse d'épaule à produire reçoit vers l'intérieur le corps d'implantation et a sa couche interne d'os spongieux qui est compactée d'une manière planifiée qui est différenciée le long et autour d'un axe d'implantation du corps d'implantation en fonction de la densité et de la quantité d'os local préopératoire de la couche interne d'os spongieux, et
une étape de fabrication, dans laquelle le corps d'implantation conçu à l'étape de conception est fabriqué.

12. Procédé selon la revendication 11, dans lequel l'étape de conception comprend les étapes consistant à :
définir des règles qui sont basées sur les données osseuses du patient et qui concernent la densité et la quantité d'os local préopératoire de la couche interne d'os spongieux (H2 ; G2) par rapport à une condition compactée souhaitée pour la couche interne d'os spongieux de l'os lorsqu'il est remplacé avec le composant de prothèse d'épaule (10 ; 20 ; 30 ; 40) à produire, et
utiliser les règles pour concevoir le corps d'implantation (11 ; 21 ; 31 ; 41).

13. Procédé selon la revendication 11 ou la revendication 12, dans lequel l'étape consistant à fournir des données comprend l'étape consistant à réaliser un balayage CT de l'os (H ; G) et segmenter les images CT du balayage CT en données osseuses du patient.

14. Procédé selon l'une quelconque des revendications 11 à 13, dans lequel l'étape de fabrication comprend l'étape consistant à utiliser un processus de fabrication additif.

15. Procédé selon l'une quelconque des revendications 11 à 14, dans lequel le procédé comprend en outre l'étape consistant à coupler le corps d'implantation (11 ; 21 ; 31 ; 41) fabriqué à l'étape de fabrication avec un élément d'articulation (14 ; 24 ; 34 ; 44) du composant de prothèse d'épaule (10 ; 20 ; 30 ; 40), l'élément d'articulation étant prévu avec une surface d'articulation (14A).
